# EUROPÄISCHE PATENTSCHRIFT

(11) **EP 3 085 323 B1**
(45) Veröffentlichungstag und Bekanntmachung des Hinweises auf die Patenterteilung: **04.10.2017**
(21) Anmeldenummer: 16165822.4
(22) Anmeldetag: 18.04.2016
(51) Int. Cl.: A61B 50/31, A61B 50/20

(54) **TRANSPORTBEHÄLTER UND AUSRÜSTUNGSMODUL FÜR MEDIZINISCHE AUSRÜSTUNG, SET HIERAUS SOWIE VERFAHREN ZUR HERSTELLUNG EINES TRANSPORTBEHÄLTERS**
TRANSPORT CONTAINER AND EQUIPMENT MODULE FOR EQUIPMENT HAVING A MEDICAL APPLICATION, SET THEREFROM AND METHOD FOR PRODUCING A TRANSPORT CONTAINER
RECIPIENT DE TRANSPORT ET MODULE D'EQUIPEMENT POUR EQUIPEMENT MEDICAL, ENSEMBLE AINSI CONSTITUE ET PROCEDE DE FABRICATION D'UN RECIPIENT DE TRANSPORT

(30) Priorität: 21.04.2015 DE 202015101973 U
(43) Veröffentlichungstag der Anmeldung: 26.10.2016
(73) Patentinhaber: HUM Gesellschaft für Homecare und Medizintechnik mbH, 44536 Lünen (DE)
(72) Erfinder: Breese, Bettina, 33161 Hövelhof (DE)
(74) Vertreter: Kalkoff & Partner

(56) Entgegenhaltungen:
- DE-U1-202014 000 119
- FR-A1- 2 936 141
- US-A- 4 068 655
- US-A1- 2014 014 544

## Beschreibung

Die Erfindung betrifft einen Transportbehälter und ein Ausrüstungsmodul für medizinische Ausrüstung, ein Set aus einem Transportbehälter und einem Ausrüstungsmodul sowie ein Verfahren zur Herstellung eines Transportbehälters.

Herkömmliche Systeme für die Aufbewahrung von medizinischer Ausrüstung, wie z. B. Notfalltaschen oder -rucksäcke für den Rettungsdienst, weisen häufig Innentaschen oder spezifische Halterungen für medizinische Ausrüstung auf, welche in variablen Positionen in dem Transportbehälter angebracht werden können. Die Fixierung dieser Innentaschen oder Halterungen im Transportbehälter erfolgt meist über Fixierflächen aus Klettverschlüssen, die an den Innentaschen und am Transportbehälter vorgesehen sind. Hierüber können diese Elemente mit dem Korpus des Transportbehälters verbunden werden. Klettverschlüsse neigen allerdings dazu, dass sie sich relativ schnell abnutzen und nur schwer zu reinigen und zu desinfizieren sind.

Ein Transportbehälter für medizinische Ausrüstung mit magnetischen Fixierflächen ist in der DE 20 2014 000 119 U1 offenbart. Der dort gezeigte Rettungsrucksack mit Ausstattungselementen weist hinter den Fixierflächen textile, vernähte Tunnel auf, in welche Magnetstreifen als Befestigungselemente eingeschoben werden können. Die Tunnel sind so gestaltet, dass für eine Reinigung die Magnetstreifen herausgenommen werden können. Um eine Verschmutzung der Nähte zu vermeiden, sind diese mit einer zusätzlichen Materiallage abgedeckt.

Es ist daher die Aufgabe der Erfindung, eine magnetische Befestigung an Fixierflächen für medizinische Ausrüstung zu ermöglichen, welche eine einfache Produktion und gute Haltbarkeit ermöglicht.

Die Aufgabe der Erfindung wird jeweils gelöst durch einen Transportbehälter gemäß Anspruch 1, durch ein Ausrüstungsmodul gemäß Anspruch 7, durch ein Set aus Transportbehälter und Ausrüstungsmodul gemäß Anspruch 11 und durch ein Verfahren gemäß Anspruch 12. Abhängige Ansprüche beziehen sich auf vorteilhafte Ausführungsformen der Aspekte der Erfindung.

Bei dem Transportbehälter kann es sich bevorzugt um einen Rucksack, Koffer oder eine Tasche handeln, wie sie bspw. im Rettungsdienst und in der Notfallmedizin zum Transport von medizinischer Ausrüstung üblich sind.

Erfindungsgemäß umfasst ein Transportbehälter für medizinische Ausrüstung mindestens eine Fixierfläche. Diese ist bevorzugt eine Innenfläche des Transportbehälters.

Die Fixierfläche weist erfindungsgemäß mindestens einen Hohlraum auf, in dem ein Magnetbefestigungselement angeordnet ist. Ein Magnetbefestigungselement ist als Gegenstand mit magnetischen Eigenschaften zu verstehen. Beispiele für Magnetbefestigungselemente wären z. B. ein Weichmagnet, bspw. in Form einer Stahlscheibe oder ein Dauermagnet, wie z. B. ein Samarium- oder Neodym-Magnet. Durch seine magnetischen Eigenschaften kann das Magnetbefestigungselement eine lösbare Verbindung mit anderen magnetischen Elementen eingehen, welche dann ein Gegenstück zum Magnetbefestigungselement bilden.

Daher kann mittels des Magnetbefestigungselements eine lösbare und dennoch sichere Anbringung von einem mit einem magnetischen Element ausgestatteten Gegenstand, wie bspw. einer entsprechenden Innentasche oder einem Ausrüstungsmodul für medizinische Ausrüstung an der Fixierfläche des Transportbehälters erfolgen.

Ausrüstungsmodule können lösbar über eine magnetische Befestigung in variablen Positionen im Transportbehälter an der Fixierfläche angebracht werden. Bei den Ausrüstungsmodulen kann es sich bspw. um Innentaschen oder aber auch sonstige medizinische Ausrüstung handeln, wie bspw. Ampullenhalter, Laringoskophalter oder Venflon-Halterungen.

Ein erfindungsgemäßes Ausrüstungsmodul umfasst mindestens eine Modulfixierfläche. Die Modulfixierfläche umfasst mindestens einen Hohlraum, in welchem ein Magnetbefestigungselement angeordnet ist. Bevorzugt ist die Modulfixierfläche eine Außenfläche des Ausrüstungsmoduls.

Ein Magnetbefestigungselement ist kann vorteilhafterweise sowohl im Transportbehälter als auch im Ausrüstungsmodul in dem jeweiligen Hohlraum in einer Ebene parallel zur Fixierfläche frei verschiebbar sein; es kann so innerhalb des jeweiligen Hohlraumes seine Position auf dieser Ebene verändern. Der Hohlraum bietet für eine Verschiebbarkeit des Magnetbefestigungselements sowohl in seiner Länge als auch in seiner Breite ausreichend Platz, so dass das Magnetbefestigungselement bspw. mindestens um eine Strecke seines halben Durchmessers entlang der Länge und Breite des Hohlraums beweglich ist. Diese Beweglichkeit des Magnetbefestigungselements hat den Vorteil, dass sich die Magnetbefestigungselemente bei einer Anbringung bspw. eines Ausrüstungsmoduls mit Magnetelementen an der Fixierfläche, durch die Magnetwirkung verschieben können. So können bei Anbringung eines Ausrüstungsmoduls an die Fixierfläche des Transportbehälters die jeweiligen Magnetbefestigungselemente innerhalb des Hohlraumes ihre Position auf der Ebene verlagern und so ein passendes Gegenstück auf der Fixierfläche des Transportbehälters, bzw. der Modulfixierfläche finden. Dadurch kann immer eine bestmögliche Haltewirkung erzielt werden und die Fixierfläche kommt, im Vergleich der Gesamtfläche, mit weniger Magnetbefestigungselementen aus. Bevorzugt ist es, wenn die Fläche, welche das Magnetbefestigungselement einnimmt, zwischen 5-60 % der Fläche des Hohlraumes beträgt, besonders bevorzugt sind beim Transportbehälter 25-60% und bei dem Ausrüstungsmodul 5-20%. Dadurch kann an magnetischem Material gespart werden.

Durch diesen Aufbau liegt das Magnetfestigungselement nicht immer an der selben Stelle. Dies führt bei Befestigung bspw. eines Ausrüstungsmoduls mit Magneten zu weniger Abrieb und einer geringeren Materialabnutzung.

Bei dem Transportbehälter, bei dem Ausrüstungsmodul sowie bevorzugt bei beiden ist der Hohlraum, in dem das Magnetbefestigungselement angeordnet ist, bspw. durch ein Vernähen oder Verschweißen zweier aufeinanderliegender Schichten flächigen Materials gebildet, bspw. durch eine Doppellage des Materials. Vorteilhaft ist es, wenn das flächige Material textil und biegsam ist, besonders vorteilhaft ist es, wenn es wasserdicht und abwischbar ist. Bevorzugt besteht mindestens eine obere Schicht des flächigen Materials aus einer PVCbeschichteten Plane. Vorteilhaft ist es, wenn eine Schweißnaht am Hohlraum wasserundurchlässig ist. Hierdurch ergibt sich trotz Unterteilung der Fixierfläche bzw. Modulfixierfläche eine geschlossene Oberfläche. Bevorzugt kann die Schweißnaht durch Ultraschallschweißen gebildet werden. Hierbei handelt es sich um eine besonders schnelle und wenig energieintensive Methode, welche luft- und flüssigkeitsdichte Schweißnähte ermöglicht. Andere Schweißtechniken, wie bspw. Laserschweißen, sind auch möglich.

Sowohl eine wasserundurchlässige Schweißnaht als auch eine PVC-beschichtete Oberfläche der Fixierfläche ermöglichen ein einfaches und schnelles Reinigen und Desinfizieren der Fixierfläche. Zudem verhindern die wasserundurchlässigen Schweißnähte und die PVC-beschichtete Oberfläche eine Kontamination unter der Fixierfläche liegender Schichten des Transportbehälters, bzw. des Inhalts des Ausrüstungsmoduls.

Es ist vorteilhaft, sowohl für den Transportbehälter als auch für das Ausrüstungselement, wenn die Fixierfläche, bzw. Modulfixierfläche eine Mehrzahl von Hohlräumen mit jeweils darin angeordneten Magnetbefestigungselementen aufweist. Besonders vorteilhaft ist es, wenn die Hohlräume matrixartig angeordnet sind. Eine solch matrixartige Anordnung kann ein Raster mit untereinander angeordneten Zeilen und nebeneinander angeordneten Spalten sein. Das Raster kann dabei als Gitternetz ausgebildet sein, wobei Gitternetzlinien den Schweißnähten entsprechen. Durch die Anordnung in einem Raster ist die Fixierfläche, bzw. Modulfixierfläche, flexibel gestaltet, so dass bspw. am Transportbehälter Ausrüstungsmodule verschiedenster Größen und Formen über eine magnetische Befestigung angebracht werden können. Außerdem bietet die matrixartige Anordnung der Hohlräume einen Vorteil beim Vernähen, bzw. Verschweißen der Nähte, da so mehrere Hohlräume durch wenige, sich bevorzugt überkreuzende Nähte gebildet werden können. Bevorzugt weist die Fixierfläche 1-9 Zeilen und 1-9 Spalten, und somit 1-81 Felder, d. h. Hohlräume, auf.

Besonders vorteilhaft ist es, wenn ein bspw. gitternetzartiges Raster der Modulfixierflächen sich in Deckung mit einem entsprechenden Raster der Fixierflächen befindet. Dies erleichtert die Platzierung und Anbringung des Ausrüstungsmoduls an der Fixierfläche des Transportbehälters.

Der Hohlraum der Fixierfläche und der Modulfixierfläche ist vorteilhafterweise rechteckig und bspw. zwischen 3 - 7 cm lang und 3 - 16 cm breit, wobei besonders bevorzugt der Hohlraum 7 cm breit und 8 cm lang ist. Die Ausgestaltung des Hohlraums mit möglichst rechtem Winkel erleichtert ein Vernähen oder Verschweißen. Es ist vorteilhaft, wenn die Hohlräume der Modulfixierfläche und der Fixierfläche die gleichen Maße aufweisen. Dies kann eine leichtere Anbringung des Ausstattungselements an der Fixierfläche des Transportbehälters ermöglichen.

Das Magnetbefestigungselement des Transportbehälters und/oder des Ausrüstungsmoduls ist bevorzugt kreisförmig und weist bspw. einen Durchmesser zwischen 0,5 - 8 cm, weiter bevorzugt 1-8 cm auf. Ein Vorteil der Kreisform ist, dass das Magnetbefestigungselement nicht mit einer Spitze oder Kante an die Naht stoßen kann. Eine eckige, z. B. quadratische oder dreieckige, Ausgestaltung könnte dazu führen, dass eine solche spitze Kante oder Ecke die Naht angreift und somit die Fixierfläche beschädigt. Bevorzugt ist das Magnetbefestigungselement des Transportbehälters größer als das des Ausrüstungsmoduls, mit einem Flächenverhältnis zwischen 2 zu 1 und 12 zu 1.

Bei den Magnetbefestigungselementen kann es sich um Scheiben handeln, welche möglichst so flach sind, dass sie ein Verrutschen in den Hohlräumen nicht behindern. Besonders beim Transportbehälter sind weichmagnetische Elemente, insbesondere Scheiben bevorzugt, wobei dauermagnetische Elemente auch möglich sind. Vorteilhaft sind Unterlegscheiben, da diese eine Kostenersparnis darstellen.

Besonders vorteilhaft ist es, wenn das Magnetbefestigungselement des Ausrüstungsmoduls ein Dauermagnet, insbesondere ein Neodym-Magnet ist. Hierdurch kann das Ausrüstungsmodul sicher und lösbar auf einer Fixierfläche des Transportbehälters angebracht werden. Außerdem ermöglichen Dauermagneten als Magnetbefestigungselement des Ausrüstungsmoduls eine Befestigung auf verschiedensten Oberflächen, mit zumindest z. T. weichmagnetischen Eigenschaften. So kann im Einsatz das Ausrüstungsmodul von Rettungskräften auch bspw. an Kfz-Karosserien, industriellen Großgeräten oder Heizkörpern und dergleichen platziert und angebracht werden.

Die Fixierfläche erstreckt sich vorteilhafterweise zumindest teilweise über mindestens einen Boden des Transportbehälters. In vorteilhaften Ausführungsformen ist noch mindestens eine zweite Fixierfläche an einem Innenrand des Transportbehälters angeordnet. Diese erstreckt sich mindestens über die gesamte Innenseite einer Seitenfläche. Die beiden Fixierflächen können so in einem Winkel von bspw. mehr als 30° zueinander stehen, wobei bevorzugt der Winkel 90° ± 10° beträgt. Dadurch können entsprechend größere und schwerere Ausrüstungsmodule im Transportbehälter sicher befestigt werden, sofern diese auch über Magnetbefestigungselemente an mehr als einer Außenseite verfügen.

Besonders vorteilhaft ist es, wenn sich eine Modulfixierfläche über eine erste Außenwand des Ausrüstungsmoduls erstreckt und eine zweite Modulfixierfläche über eine zweite Außenwand erstreckt. Ähnlich wie die entsprechenden Fixierflächen eines Transportbehälters stehen die beiden Modulfixierflächen in einem Winkel zueinander, welcher vorteilhafterweise auch 90° ± 10° ist. Mittels dieser beiden Modulfixierflächen können größere und schwerere Ausrüstungsmodule an den Fixierflächen einer vorteilhaften Ausführungsform des Transportbehälters angebracht werden. Außerdem kann im Einsatz das Ausrüstungsmodul durch diese Modulfixierfläche in einer variablen Position angebracht werden.

Am Transportbehälter können mehrere Fixierflächen bevorzugt sein, welche sich an zwei Seiten des Transportbehälters gegenüberliegen, bspw. wenn dieser ein aufklappbarer Rucksack ist, oder eine Fixierfläche sich im Deckel eines Koffers befindet. So können bspw. mehr verschiedene Ausrüstungsmodule kleineren Volumens fixiert und transportiert werden.

Vorteilhaft ist, wenn neben den beiden Schichten, welche die Fixierfläche mit den Hohlräumen bilden, der Transportbehälter noch weitere Schichten umfasst. Zur Formgebung des Korpus ist es vorteilhaft, wenn ein formstabiles Element eine Unterlage bildet, auf der bzw. an der andere Schichten angebracht werden können. Diese formstabile Schicht bildet somit einen Grundkörper und gibt die Form des Korpus vor. Bevorzugt ist dieses stabile Element eine PE-Platte. Dadurch ist die formgebende Unterlage leicht, stabil und kostengünstig.

Vorteilhaft ist es, wenn an der formgebenden Unterlage eine komprimierbare Schicht, bspw. PE-Schaum, nach außen angebracht ist. Diese eindrückbare Isolierschicht isoliert das Innere des Transportbehälters und kann eventuellen Stoßschäden vorbeugen. Einen Abschluss des Korpus nach außen bildet eine wasserabweisende Schicht, bspw. aus PVC-beschichteter Plane. Dadurch wird der Inhalt des Transportbehälters vor Witterungseinflüssen geschützt und die Außenseite ist auch leicht abwaschbar.

Das Ausrüstungsmodul kann in bevorzugten Ausführungsformen, außer im Bereich der beiden Schichten welche die Fixierfläche mit den Hohlräumen bilden, nur aus einer Materiallage bestehen, vorteilhafterweise aus dem gleichen Material wie die äußere Schicht der Fixierfläche, bspw. PVC-beschichteter Plane. So kann das Ausrüstungsmodul kostengünstig hergestellt werden, ist leicht zu reinigen und weist ein geringes Gewicht auf.

Alternative Ausführungsformen des Ausrüstungsmoduls können weitere Materiallagen umfassen. Bei den weiteren Materiallagen kann es sich z. B. um polsternde Schichten, bspw. aus PE-Schaum, formgebende Lagen, wie bspw. PE-Platten und/oder weiteren Lagen PVC-beschichteter Plane, handeln. Das Ausrüstungsmodul kann daher bei der Herstellung entsprechend verschiedener Bedürfnisse, z.B. für einen gewissen Schutz des Inhalts gegenüber Stößen, ausgestaltet werden.

Bevorzugt kann ein Transportbehälter ein oder mehrere Ausrüstungsmodule aufnehmen. Vorteilhaft ist es, wenn ein Ausrüstungsmodul an einer Fixierfläche des Transportbehälters angeordnet ist, wobei es besonders vorteilhaft ist, wenn die Modulfixierfläche überdeckend zur Fixierfläche angeordnet ist. Bevorzugt befindet sich hierbei ein Raster der Modulfixierfläche in Deckung mit einem Raster der Fixierfläche.

Nachfolgend wird eine Ausführungsform der Erfindung anhand von Zeichnungen näher beschrieben. Dabei zeigen:
- Fig. 1: eine perspektivische Ansicht eines Transportbehälters und eines Ausrüstungsmoduls;
- Fig. 2: eine Draufsicht auf eine Fixierfläche des Transportbehälters;
- Fig. 3: eine perspektivische Ansicht eines Ausrüstungsmoduls;
- Fig. 4: eine Draufsicht auf eine Modulfixierfläche eines Ausrüstungsmoduls;
- Fig. 5: ein Querschnitt durch den Korpus des Transportbehälters und des Ausrüstungsmoduls.

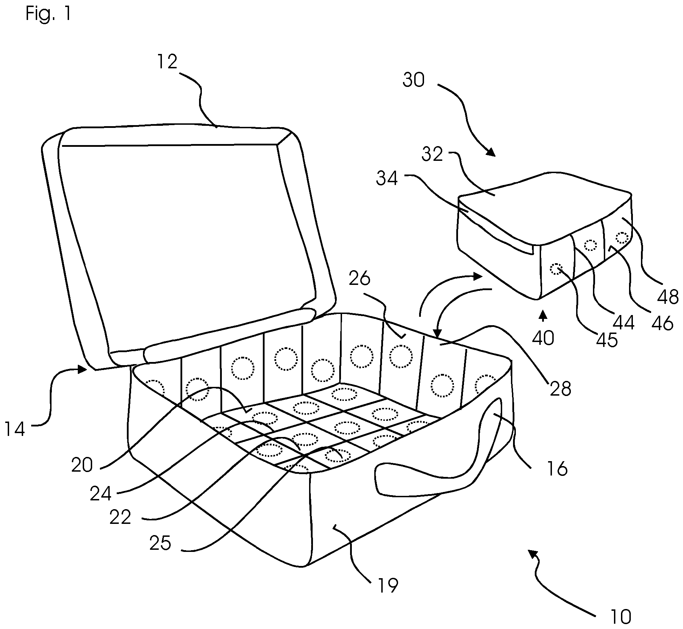

Fig. 1 zeigt eine perspektivische Ansicht eines Transportbehälters 10 für medizinische Ausrüstung, im Weiteren Koffer 10 genannt, und eines Ausrüstungsmoduls 30. Bei dem Koffer 10 kann es sich um einen Notarztkoffer, oder ähnliches, handeln.

Der Koffer 10 weist an seiner Außenseite eine wasserabweisende textile Schicht aus PVC-beschichteter Plane 19 auf. Er weist einen Deckel 12 auf, welcher mit dem Grundkörper des Koffers 10 über ein textiles Scharnier 14 verbunden ist. Über einen Reißverschluss ist der Koffer 10 verschließbar und mittels eines am Koffer 10 angebrachten Henkels 16 kann er transportiert werden. Der Koffer 10 weist in seinem Inneren eine mit einem Gitternetz-Muster gerasterte Fixierfläche 20 auf. Die einzelnen Rasterelemente sind Hohlräume 22, in denen als Magnetbefestigungselemente weichmagnetische Scheiben 25 angeordnet sind. Die Linien des Rasters sind gebildet durch überkreuzend verlaufende Schweißnähte 24.

Das Ausrüstungsmodul 30 ist eine Innentasche für den Koffer 10, in welcher medizinische Ausrüstung aufbewahrt werden kann. Das Ausrüstungsmodul 30 kann flexibel mit verschiedenster medizinischer Ausrüstung befüllt werden. In alternativen Ausführungsformen ist das Ausrüstungsmodul 30 bspw. eine Ampullenhalterung oder andere Halterung für medizinische Ausrüstung.

Das Ausrüstungsmodul 30 weist einen Deckel 32 und zum Verschließen einen Reißverschluss 34 auf. An jeweils einer Außenseite, nämlich einer Unterseite und einer Längsseite, sind außen jeweils eine Modulfixierfläche 40 (unten; in Fig. 1 nicht sichtbar) und 46 (längs) angeordnet. Diese Modulfixierflächen sind wie die Fixierflächen des Koffers 10 gerastert. Die einzelnen Rasterelemente sind auch hier Hohlräume 42, gebildet durch Schweißnähte 44. In den Hohlräumen 44 befindet sich jeweils ein Magnetbefestigungselement, ein Neodym-Magnet, im Folgenden Magnet 45 genannt. Die Raster der Modulfixierflächen 40 und 46 sind passend zu den Fixierflächen 20, 26 des Koffers 10 gestaltet.

Sowohl die Scheiben 25 als auch die Magnete 45 sind in ihren jeweiligen Hohlräumen 22 und 42, bzw. 28 und 48 frei verschiebbar innerhalb der jeweiligen Ebene der Fixierfläche bzw. Modulfixierfläche. Die Scheiben 25 und Magnete 45 können dadurch ihre Position innerhalb eines Hohlraumes 22 verändern.

Über die magnetische Befestigung kann das Ausrüstungsmodul 30 lösbar in einer frei wählbaren und variablen Position innerhalb des Koffers 10 fixiert werden.

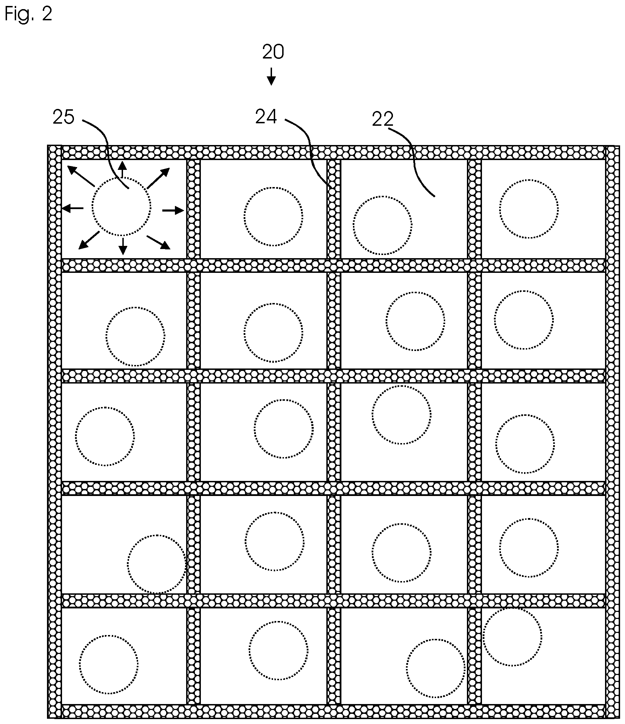

In der Fig. 2 ist die Fixierfläche 20 des Koffers 10 in einer Draufsicht gezeigt. Die Hohlräume 22 sind in einer matrixartigen Rasterung angeordnet. Die Hohlräume 22 sind gebildet durch das Verschweißen zweier aufeinanderliegender PVC-beschichteter Planen 21. In den Hohlräumen 22 befinden sich als magnetische Befestigungselemente jeweils metallische Scheiben 25, welche innerhalb des jeweiligen Hohlraumes 22 frei beweglich und verschiebbar sind.

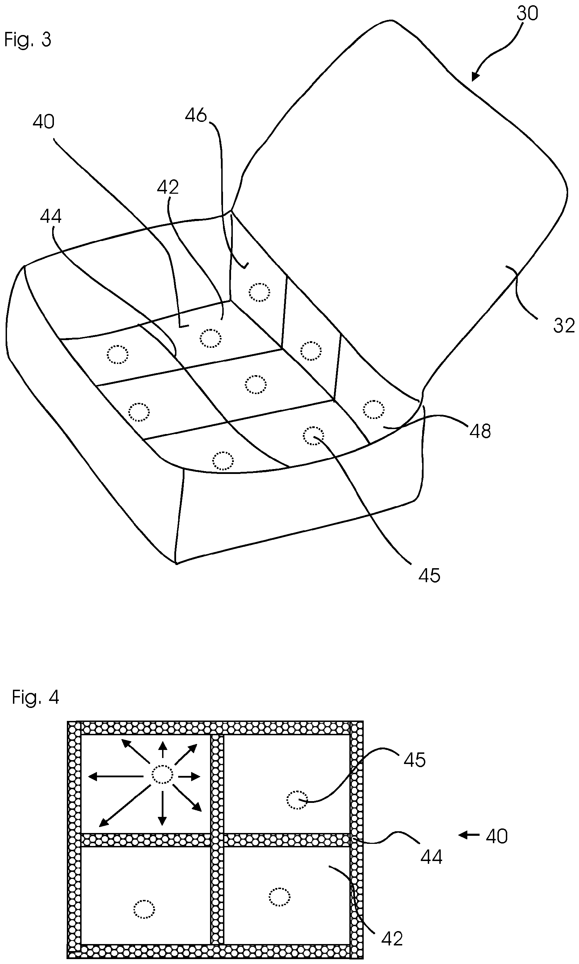

Der Korpus des Ausrüstungsmoduls 30, gezeigt in Figur 3, besteht nur aus zwei Lagen PVC-beschichteter Plane. Daher ist das Raster der Fixierfläche 40 auch gut im geöffneten Zustand des Ausrüstungsmoduls 30 sichtbar. In der gezeigten Ausführungsform umfasst die Fixierfläche 40 eines Bodens 3 x 2 Hohlräume 42, in welchen Magnete 45 eingesetzt sind. In alternativen Ausführungsformen kann diese Matrix auch entsprechend größer oder kleiner ausfallen. Über die hintere Seitenwand erstreckt sich eine Modulfixierfläche 46. Diese weist in der gezeigten Ausführungsform 3 x 1 Hohlräumen 48 auf. In alternativen Ausführungsformen eines Ausrüstungsmoduls 30 kann dieses matrixartige Raster auch entsprechend größer oder kleiner ausfallen, bzw. die Modulfixierfläche 46 kann auch ganz entfallen.

Die Modulfixierfläche 46, gezeigt in Fig. 4, weist Hohlräume 42 auf, in welchen magnetische Befestigungselemente angeordnet sind. Bei diesen handelt es sich um dauermagnetische Scheiben in Form von Neodym-Magneten 45. Die Schweißnähte 44 sind entlang der Gitternetzlinien eines Rasters gebildet und grenzen so die Hohlräume 42 voneinander ab. Wie die Scheiben 25 der Fixierfläche 20 des Koffers, so sind auch die Magnete 45 in ihren jeweiligen Hohlräumen 22 frei auf einer Ebene beweglich.

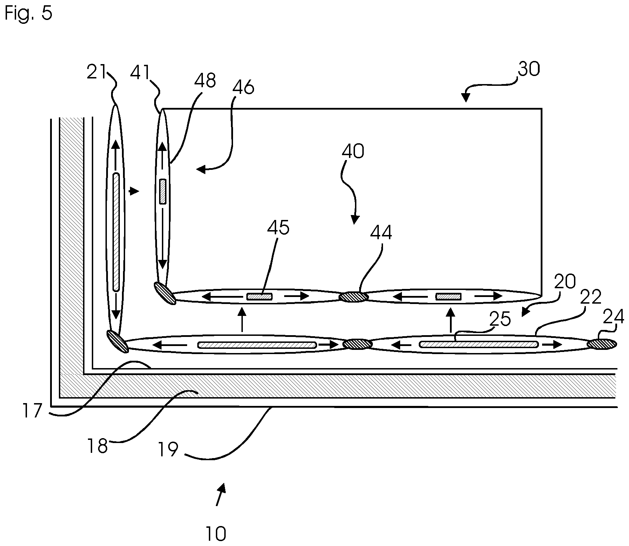

Der Korpus des Koffers 10, gezeigt in Fig. 5, besteht in der gezeigten Ausführungsform aus fünf Schichten. Die äußerste Schicht 19 ist eine wasserabweisende PVC-beschichtete Plane. Auf diese folgt nach innen ein komprimierbarer PE-Schaum 18. Dieser Schaum 18 kann Stöße abfedern und wirkt zudem isolierend auf die Inhalte des Koffers 10. Angebracht ist dieser Schaum 18 an einer formstabilen PE-Platte 17. Diese gibt dem Koffer seine Form und bildet eine stabile Grundlage für die inneren Schichten. Auf der Innenseite des Koffers 10 ist an der PE-Platte 17 die Fixierfläche 20 angebracht. Die Fixierfläche 20 ist gebildet aus aufeinanderliegenden Lagen flächigen Materials, hier PVC-beschichtete Plane 21. Diese Planen 21 sind miteinander über Schweißnähte 24 so verbunden, dass sich zwischen ihnen Hohlräume 22 bilden, in welcher die Scheiben 25 angeordnet sind.

Eine zweite Fixierfläche 26 ist an einem Innenrand des Koffers 10 angebracht und steht in einem rechten Winkel zur ersten Fixierfläche 20 am Boden des Koffers 10.

Das Ausrüstungsmodul 30 besteht vollständig bis auf einen Reißverschluss aus PVC-beschichteter Plane. Im Bereich die Modulfixierflächen 40 und 46 ist diese Plane doppellagig angeordnet. Das Ausrüstungsmodul 30 kann in alternativen Ausführungsformen auch mehrere Materiallagen, wie bspw. PE-Platten, umfassen und kann ggf. auch gepolstert sein, z.B. durch eine Lage PE-Schaum. Diese aufeinanderliegenden Lagen 41 sind über Schweißnähte 44 miteinander verbunden, so dass auch zwischen ihnen sich Hohlräume bilden, in welche Magnete 45 eingelegt sind.

Der Vorteil der Verschiebbarkeit der Magnete 45 und Scheiben 25 ist, wie Fig. 5 zeigt, dass bei Fixierung des Ausrüstungsmoduls 30 im Koffer 10 Fixiereinheiten, also Magnet 45 und Scheibe 25, sich so gegeneinander verschieben können, dass ein guter Halt gegeben ist. Durch die Verschiebung der Position auf einer Ebene können sie sich so zueinander verlagern, dass die Magnete 45 möglichst zentral auf den Scheiben 25 stehen. Dies begünstigt einen stabilen Halt des Ausrüstungsmoduls 30 im Koffer 10 an den Fixierflächen 20 und 26.

## Patentansprüche

1. Transportbehälter (10) für medizinische Ausrüstung, umfassend
- mindestens eine Fixierfläche (20), wobei die Fixierfläche (20) mindestens einen Hohlraum (22) aufweist,
- und ein Magnetbefestigungselement (25), das in dem Hohlraum (22) angeordnet ist und in dem Hohlraum (22) in einer Ebene parallel zur Fixierfläche (20) frei verschiebbar ist.

2. Transportbehälter (10) für medizinische Ausrüstung nach Anspruch 1, wobei der Hohlraum (22) gebildet ist durch ein Vernähen oder Verschweißen zweier aufeinanderliegender Schichten (21) flächigen Materials.

3. Transportbehälter (10) für medizinische Ausrüstung nach Anspruch 2, wobei eine Schweißnaht (24) am Hohlraum (22) wasserundurchlässig ist.

4. Transportbehälter (10) für medizinische Ausrüstung nach einem der vorhergehenden Ansprüche, wobei die Fixierfläche (20) eine Mehrzahl von matrixartig angeordneten Hohlräumen (22) und darin angeordneten Magnetbefestigungselementen (25) aufweist.

5. Transportbehälter (10) für medizinische Ausrüstung nach einem der vorhergehenden Ansprüche, wobei die Fixierfläche (20) sich zumindest teilweise über einen Boden des Transportbehälters (10) erstreckt, und eine zweite Fixierfläche (26) an einem Innenrand des Transportbehälters (10) angeordnet ist, so dass die zweite Fixierfläche (26) in einem Winkel zur ersten Fixierfläche (20) angeordnet ist.

6. Transportbehälter (10) für medizinische Ausrüstung nach einem der vorhergehenden Ansprüche, wobei ein Korpus des Transportbehälters (10) mehrere Lagen umfasst, wobei im Transportbehälter (10) mindestens zwei Schichten aus einem flächigen wasserabweisenden Material, welche die Fixierfläche (20) mit den Hohlräumen (22) bilden, angeordnet sind, ein formstabiles Element eine Unterlage (17) bildet, auf der diese beiden Schichten angebracht sind, wobei nach außen an der Unterlage eine Lage komprimierbaren Materials (18) angebracht ist und die äußere Schicht des Korpus aus einer wasserabweisenden Schicht (19) besteht.

7. Ausrüstungsmodul (30) für medizinische Ausrüstung, umfassend mindestens eine Modulfixierfläche, wobei die Modulfixierfläche (40) mindestens einen Hohlraum (42) umfasst, in dem ein Magnetbefestigungselement angeordnet ist und wobei das Magnetbefestigungselement (45) in einer Ebene parallel zur Modulfixierfläche (40) frei verschiebbar ist.

8. Ausrüstungsmodul (30) für medizinische Ausrüstung gemäß Anspruch 7, wobei die Modulfixierfläche (40) an einer Außenseite des Ausrüstungsmoduls (30) angeordnet ist und eine Mehrzahl von Hohlräumen (42) und Magnetbefestigungselementen (45) aufweist, wobei die Hohlräume (42) matrixartig angeordnet sind.

9. Ausrüstungsmodul (30) für medizinische Ausrüstung gemäß einem der Ansprüche 7, 8, wobei das Magnetbefestigungselement ein Dauermagnet (45) ist.

10. Ausrüstungsmodul (30) für medizinische Ausrüstung gemäß einem der Ansprüche 7 bis 9, wobei sich eine Modulfixierfläche über eine erste Außenwand des Ausrüstungsmoduls (30) und eine zweite Modulfixierfläche (46) über eine zweite Außenwand erstreckt, so dass die zweite Modulfixierfläche (46) in einem Winkel zur ersten Modulfixierfläche (40) steht.

11. Set aus einem Transportbehälter (10) gemäß einem der Ansprüche 1 bis 6 und mindestens einem Ausrüstungsmodul (30) gemäß einem der Ansprüche 7-10, wobei ein Raster der Modulfixierfläche (40) sich in Deckung mit einem Raster der Fixierfläche (20) befindet.

12. Verfahren zur Herstellung eines Transportbehälters (10) für medizinische Ausrüstung, bei dem
- durch Vernähen oder Verschweißen zweier aufeinanderliegender Schichten (21) flächigen Materials in einer Fixierfläche (20) des Transportbehälters (10) eine Anzahl von matrixartig angeordneten Hohlräumen (22) gebildet werden,
- und Magnetbefestigungselemente (25) in den Hohlräumen (22) so angeordnet werden, dass sie jeweils in den Hohlräumen (22) in einer Ebene parallel zur Fixierfläche (20) frei verschiebbar sind.

## Claims

1. A transport container (10) for equipment having a medical application, comprising
- at least one fixing surface (20), wherein the fixing surface (20) has at least one hollow space (22),
- and a magnetic fixing element (25), which is arranged in the hollow space (22) and is freely shiftable in the hollow space (22) in a plane parallel to the fixing surface (20).

2. The transport container (10) for equipment having a medical application according to claim 1, wherein the hollow space (22) is formed by a sewing or welding of two plies (21) of sheet material lying on top of each other.

3. The transport container (10) for equipment having a medical application according to claim 2, wherein a welded seam (24) on the hollow space (22) is water-impermeable.

4. The transport container (10) for equipment having a medical application according to one of the preceding claims, wherein the fixing surface (20) has a plurality of hollow spaces (22) arranged in a matrix-like manner and magnetic fixing elements (25) arranged therein.

5. The transport container (10) for equipment having a medical application according to one of the preceding claims, wherein the fixing surface (20) extends at least partially over a floor of the transport container (10), and a second fixing surface (26) is arranged on an inner edge of the transport container (10) so that the second fixing surface (26) is arranged at an angle to the first fixing surface (20).

6. The transport container (10) for equipment having a medical application according to one of the preceding claims, wherein a corpus of the transport container (10) comprises several layers, wherein at least two plies of a laminar, water-repellant material, which form the fixing surface (20) with the hollow spaces (22), are arranged in the transport container (10); a dimensionally stable element forms an underlay (17), to which these two plies are applied, wherein a layer of compressible material (18) is applied towards the outside on the underlay; and the outer ply of the corpus is made of a water-repellant ply (19).

7. An equipment module (30) for equipment having a medical application, comprising at least one module fixing surface, wherein the module fixing surface (40) comprises at least one hollow space (42), in which a magnetic fixing element is arranged, and wherein the magnetic fixing element (45) is freely shiftable in a plane parallel to the module fixing surface (40).

8. The equipment module (30) for equipment having a medical application according to claim 7, wherein the module fixing surface (40) is arranged on an outside of the equipment module (30) and has a plurality of hollow spaces (42) and magnetic fixing elements (45), wherein the hollow spaces (42) are arranged in a matrix-like manner.

9. The equipment module (30) for equipment having a medical application according to one of claims 7, 8, wherein the magnetic fixing element is a permanent magnet (45).

10. The equipment module (30) for equipment having a medical application according to one of claims 7 to 9, wherein a module fixing surface extends over a first outer wall of the equipment module (30) and a second module fixing surface (46) over a second outer wall so that the second module fixing surface (46) is at an angle to the first module fixing surface (40).

11. A set from a transport container (10) according to one of claims 1 to 6 and at least one equipment module (30) according to one of claims 7-10, wherein a raster of the module fixing surface (40) coincides with a raster of the fixing surface (20).

12. A method for producing a transport container (10) for equipment having a medical application, in which
- a number of hollow spaces (22) arranged in a matrix-like manner are formed by the sewing or welding of two plies (21) of sheet material lying on top of each other in a fixing surface (20) of the transport container (10),
- and magnetic fixing elements (25) are arranged in the hollow spaces (22) such that they are each freely shiftable in the hollow spaces (22) in a plane parallel to the fixing surface (20).

## Revendications

1. Récipient de transport (10) pour équipement médical, comportant :
- au moins une surface de fixation (20), la surface de fixation (20) présentant au moins un espace creux (22),
- et un élément de fixation d'aimant (25) disposé dans l'espace creux (22) et déplaçable librement dans l'espace creux (22) dans un plan parallèle à la surface de fixation (20).

2. Récipient de transport (10) pour équipement médical selon la revendication 1, dans lequel l'espace creux (22) est formé par une couture ou une soudure de deux épaisseurs (21) de matériau plat superposées.

3. Récipient de transport (10) pour équipement médical selon la revendication 2, dans lequel une ligne de soudure (24) sur l'espace creux (22) est étanche à l'eau.

4. Récipient de transport (10) pour équipement médical selon l'une des revendications précédentes, dans lequel la surface de fixation (20) présente une pluralité d'espaces creux (22) disposés à la façon d'une matrice et des éléments de fixation d'aimant (25) disposés dans ceux-ci.

5. Récipient de transport (10) pour équipement médical selon l'une des revendications précédentes, dans lequel la surface de fixation (20) s'étend au moins partiellement sur un fond du récipient de transport (10), et une deuxième surface de fixation (26) est disposée sur un bord intérieur du récipient de transport (10), de telle façon que la deuxième surface de fixation (26) est disposée dans un angle par rapport à la première surface de fixation (20).

6. Récipient de transport (10) pour équipement médical selon l'une des revendications précédentes, dans lequel un corps du récipient de transport (10) comporte plusieurs couches, dans lequel au moins deux épaisseurs constituées d'un matériau hydrophobe plat, formant la surface de fixation (20) avec les espaces creux (22), sont disposées dans le récipient de transport (10), un élément dimensionnellement stable forme une sous-couche (17) sur laquelle sont fixées les deux épaisseurs, une couche de matériau compressible (18) étant fixée vers l'extérieur sur la sous-couche, et l'épaisseur extérieure du corps étant constituée d'une épaisseur hydrophobe (19).

7. Module d'équipement (30) pour équipement médical, comportant au moins une surface de fixation de module, la surface de fixation de module (40) comportant au moins un espace creux (42) dans lequel est disposé un élément de fixation d'aimant, et l'élément de fixation d'aimant (45) pouvant être déplacé librement dans un plan parallèle à la surface de fixation de module (40).

8. Module d'équipement (30) pour équipement médical selon la revendication 7, dans lequel la surface de fixation de module (40) est disposée sur un côté extérieur du module d'équipement (30) et présente une pluralité d'espaces creux (42) et d'éléments de fixation d'aimant (45), les espaces creux (42) étant disposés à la façon d'une matrice.

9. Module d'équipement (30) pour équipement médical selon l'une des revendications 7, 8, dans lequel l'élément de fixation d'aimant est un aimant permanent (45).

10. Module d'équipement (30) pour équipement médical selon l'une des revendications 7 à 9, dans lequel une surface de fixation de module s'étend sur une première paroi extérieure du module d'équipement (30) et une deuxième surface de fixation de module (46) s'étend sur une deuxième paroi extérieure, de telle façon que la deuxième surface de fixation de module (46) forme un angle par rapport à la première surface de fixation de module (40).

11. Kit composé d'un récipient de transport (10) selon l'une des revendications 1 à 6 et d'au moins un module d'équipement (30) selon l'une des revendications 7 à 10, dans lequel une trame de la surface de fixation de module (40) coïncide avec une trame de la surface de fixation (20).

12. Procédé de fabrication d'un récipient de transport (10) pour équipement médical, dans lequel
- un nombre d'espaces creux (22) disposés à la façon d'une matrice sont formés dans une surface de fixation (20) du récipient de transport (10), par couture ou soudure de deux épaisseurs (21) de matériau plat superposées,
- et des éléments de fixation d'aimant (25) sont disposés dans les espaces creux (22) de manière à pouvoir être déplacés respectivement librement dans les espaces creux (22) dans un plan parallèle à la surface de fixation (20).
